# EUROPEAN PATENT SPECIFICATION

(11) **EP 1 303 257 B1**
(45) Date of publication and mention of the grant of the patent: **10.08.2005**
(21) Application number: 01969530.3
(22) Date of filing: 27.07.2001
(51) Int. Cl.: A61K 9/00

(54) **Method of preparing a liquid unit dosage form and a kit**
Verfahren zur Herstellung einer flüssigen Dosierungseinheit und Kit
Méthode de préparation d'une unité de dosage liquide et kit

(30) Priority: 27.07.2000 GB 0018322
(43) Date of publication of application: 23.04.2003
(73) Proprietor: The Boots Company PLC, Nottingham, Nottinghamshire NG2 3AA (GB)
(72) Inventor: KEATING, David, Anthony, Nottingham, Nottinghamshire NG2 3AA (GB); NORMAN, Kurt, Nottingham, Nottinghamshire NG2 3AA (GB)
(74) Representative: Jones, Stephen Anthony
(86) International application number: PCT/EP2001/008688
(87) International publication number: WO 2002/009666

(56) References cited:
- EP-A- 0 247 980
- EP-A- 0 552 105
- EP-A- 0 597 414

## Description

The present invention relates to pharmaceutical compositions.

In order to make medicines palatable and to make it easier for patients, for example the aged, infirm or children, who have difficulty in swallowing the usual solid dosage forms to take their medicine, it has been common practice for formulate medicines as a thickened or gelled liquid. This type of medicine is not, however, totally satisfactory for several reasons. These include:
1. great care must be taken to ensure that the formulations are stable and that there is no reduction in potency on storage;
2. the flavouring system will be one chosen by the manufacturer of the formulation and it may not be acceptable to all patients. Indeed some patients may be so deterred by the flavour chosen by the manufacturer that they will refuse to take their medicines at all;
3. active ingredients found in formulations can make the preparation taste unpleasant and bitter;
4. if the illness being treated is short in duration, then the patient may not need all the pharmaceutical composition in the container. The remainder will then either be discarded which is wasteful or will be stored, often in less-than-ideal conditions until the same illness recurs. By that time the formulation may have degraded so that it is less effective in treating the illness.
5. combination products can be difficult and lengthy to develop, in some combination products global acceptability is often not applicable; and
6. pharmacists find it difficult to prescribe combination products.

EP 552105 discloses a receptacle containing a liquid product provided with a closure lid containing a reservoir of additional liquid. The additional liquid may be added to the liquid product by manual actuation of the closure lid which causes the isolating member containing the reservoir in the lid to rupture.

EP 597414 addresses the problem of the instability of sennosides as active substances in aqueous solutions. It discloses a package comprising a granulate containing senna extract concentrate and sucrose and a bottle containing a liquid carrier. The granulate is shaken with the liquid prior to use.

EP 247980 relates to the problem of the instability of iron salts in liquid preparations. It discloses that the iron salts are stored in one container and a sweetened, flavoured carrier is contained in a separate container. Immediately before administration, the iron salt is transferred to the carrier and mixed therewith.

The present invention provides a method of preparing a liquid unit dosage form of a medicament or medicaments comprising providing a range of first containers containing different liquid carriers, providing one or more second, sealed containers containing a solid or liquid unit dose of the medicament or medicaments, pouring a quantity of a selected one of the liquid carriers into a mixing vessel, opening the one or more second, sealed containers, pouring the unit dose of the medicament or medicaments from the one or more second containers into the mixing vessel, and allowing or causing the liquid carrier and the unit dose of medicament or medicaments to become mixed in the mixing vessel. The medicament is contained as either a single unit dose or as several unit doses which can be dispensed separately.

It is envisaged that a range of liquid carriers will be available having different flavours or no flavour. It will therefore be possible to choose the flavour which is most acceptable to the patient. By choosing an acceptable tasting carrier, the patient will be more likely to take the medicine and so much better patient compliance can be anticipated. The liquid carrier may contain water and glycerol or sorbitol to provide a satisfactory mouthfeel. The amount of glycerol or sorbitol may be from 5 to 20%, preferably 8 to 15% by volume of the liquid carrier. A natural or synthetic sweetener may be included. Natural sweeteners include glucose, sucrose or maltitol. Artificial sweeteners include saccharin and pharmaceutically acceptable salts thereof, acesulphame or aspartame. The flavouring may be any of the commercially available flavourings which would be acceptable to the patient. An organic acid, for example citric acid, may be present as a flavour enhancer. The liquid carrier may be thickened with any of the known pharmaceutically acceptable thickening agents. Suitable thickening agents include starch, sodium carboxymethyl cellulose, hydroxypropyl methyl cellulose, microcrystalline cellulose, gums (such as tragacanth, acacia, carrageenan and xanthan gums), pectin, gelatin, polyethyleneglycol and partially neutralised carbomers. Preservatives, such as sodium benzoate, parabens or bronopol may be included to ensure that the liquid carrier has an acceptable shelf life. As the liquid carrier does not contain any medicaments the time consuming and expensive stability trials, which are usually needed during the development of a pharmaceutical composition to ensure that there are no undesirable interactions between any particular medicament and the carrier, are not required.

The liquid carrier may be contained in any suitable container made, for example, of glass or synthetic polymeric material. Because the contents of the container do not contain any active medicament, there is no necessity to have a so-called "child-resistant" closure on the container. These closures, which are intended to make it difficult for children to open and consume medicines accidentally, can be difficult for the aged and infirm to open. The fact that they are not needed is therefore of significant benefit to this class of patient.

The one or more second parts of the formulation may be a unit dose of the pure medicament in solid form or liquid form. This may be combined with other pharmaceutical acceptable ingredients. Alternatively a multiple dose of the medicament in a solid or liquid form, this may be pure or combined with other pharmaceutical acceptable ingredients. The other pharmaceutically acceptable ingredients may comprise 0.1% to 99.9% of the unit dose. Suitable other ingredients include taste masking agents, diluents, binders, lubricants, sweeteners and flavours.

The active medicament may be an analgesic or anti-inflammatory, decongestant, cough suppressant, expectorant, antihistamine, antiallergy agent, agent for treating the gastrointestinal tract (for example antacid, antireflux agent, antiulcer agent, antidiarrhoeal agent, laxative or antiemetic), agent to counter motion sickness, antiviral agents, antifungal agents, antibacterial agents, diuretic agents, antiasthmatic agents, antimigraine agents, vitamins and/or minerals.

Suitable analgesics include aspirin, paracetamol (acetaminophen) and non-steroidal anti-inflammatory/analgesics such as diclofenac, indomethacin, mefanamic acid, nabumetone, tolmetin, piroxicam, felbinac, diflunisal, ibuprofen, flurbiprofen, naproxen and ketoprofen or pharmaceutically acceptable salts thereof (for example the sodium or lysine salts) or narcotic analgesics such as codeine and pharmaceutically acceptable salts thereof (for example codeine, phosphate or sulphate). Caffeine may be present in analgesic products to enhance the analgesic effect.

The amount of aspirin in a unit dose may be in the range 75 to 800 mg, preferably 200-600 mg, most preferably 75, 150, 300, 400 or 600 mg. The amount of paracetamol in a unit dose may be 50 to 2000 mg, preferably 120 to 1000 mg, most preferably 120, 250, 500 or 1000 mg. The amount of diclofenac in a unit dose may be 10 to 100 mg, preferably 20 to 80 mg, most preferably 25 or 50 mg. The amount of indomethacin in a unit dose may be in the range 25-75 mg, preferably 25 mg, 50 mg or 75 mg. The amount of mefanamic acid in a unit dose may be in the range 250-500 mg, preferably 250 mg or 500 mg. The amount of nabutmetone in a unit dose may be in the range 500-1000 mg. The amount of piroxicam in a unit dose may be in the range 10-40 mg, preferably 10, 20 or 40 mg. The amount of diflunisal in a unit dose may be in the range 250-500 mg, preferably 250 mg or 500 mg. The amount of ibuprofen in a unit dose may be in the range 50 to 800 mg, preferably 100 to 400 mg, most preferably 100, 200 or 400 mg. The amount of flurbiprofen in a unit dose may be 25 to 200 mg, preferably 50 to 150 mg, most preferably 50 or 100 mg. The amount of naproxen in a unit dose may be 100 to 800 mg, preferably 200 to 600 mg, most preferably 250, 373 or 500 mg. The amount of ketoprofen in a unit dose may be 25 to 250 mg, preferably 50 to 150 mg, most preferably 50 or 100 mg. The amount of codeine in a unit dose may be 20 to 50 mg, preferably 5 to 30 mg, most preferably 8, 12.5, 16 or 25 mg. If pharmaceutically effective salts of the above compounds are used then the amount of salt should be increased to give a dose of the free medicament corresponding to the figures given above. The amount of caffeine in a unit dose may be 5 to 200 mg, preferably 10 to 100 mg, most preferably 30, 45, 60 or 100 mg.

Suitable decongestants include ephedrine, levomethol, pseudoephedrine preferably as its hydrochloride, phenylpropanolamine preferably as its hydrochloride and phenylephrine.

The amount of ephedrine in a unit dose may be in the range 15-60 mg. The amount of levomethol in a unit dose may be in the range 0.5-100 mg, preferably 0.5-25 mg, most preferably 1, 2, 5, 10 or 25 mg. The amount of pseudoephedrine preferably as its hydrochloride in a unit dose may be in the range 60-120 mg, preferably 30, 60 or 120 mg. The amount of phenylpropanolamine preferably as its hydrochloride in a unit dose may be in the range 5-50 mg, preferably 5-20 mg. The amount of phenylephrine in a unit dose may be in the range 5-25 mg, preferably 5, 10 or 25 mg.

Suitable cough suppressants include bibenzonium preferably as its bromide, caramiphen, carbetapentane preferably as its citrate, codeine, dextromethorphan preferably as its hydrobromide, noscapine and pholcodeine.

The amount of bibenzonium bromide in a unit dose may be in the range 20-30 mg. The amount of caramiphen in a unit dose may be in the range 5-20 mg, preferably 5 or 20 mg. The amount of carbetapentane citrate in a unit dose may be in the range 15-30 mg. The amount of codeine in a unit dose maybe in the range 2-50 mg, preferably 5-30mg, most preferably 10 mg. In the present invention pharmaceutically acceptable salts of codeine may also be used (for example codeine phosphate or sulphate). The amount of dextromethorphan hydrobromide in a unit dose may be in the range 15-30 mg, preferably 15 or 30 mg. The amount of noscapine in a unit dose may be in the range 15-30 mg. The amount of pholcodeine in a unit dose may be in the range 2-25 mg, preferably 5 to 20 mg, more preferably 10 to 15 mg.

Suitable expectorants include ammonium bicarbonate, ammonium chloride, bromhexine hydrochloride, cocillana creosote, guaifenesin, ipecacuanha, potassium and pharmaceutically acceptable salts thereof (for example potassium citrate or iodide), potassium guaicolsulfonate, squill and terpin hydrate.

The amount of ammonium bicarbonate in a unit dose may be in the range 300-600 mg. The amount of ammonium chloridein in a unit dose may be in the range 0.3-2 g (300-2000 mg). The amount of bromhexine hydrochloride in a unit dose may be in the range 24-64 mg. The amount of creosote in a unit dose may be in the range 0.12-0.6 ml. The amount of guaifenesin in a unit dose may be in the range 100-200 mg, preferably 100 mg. The amount of ipecacuanha in a unit dose may be in the range 25-100 mg. The amount of potassium iodide in a unit dose may be in the range 150-300 mg, preferably 100 mg. The amount of potassium citrate in a unit dose may be in the range 150-300 mg, preferably 100 mg. The amount of potassium guericolsulfonate in a unit dose may be 80 mg. The amount of squill in a unit dose may be in the range 60-200 mg. The amount of terpin hydrate in a unit dose may be in the range 125-600 mg, preferably 300 mg.

Suitable antihistamines include azatadine or a salt thereof such as the maleate, bromodiphenhydramine or a salt thereof such as the hydrochloride, brompheniramine or a salt thereof such as the maleate, carbinoxamine or a salt thereof such as the maleate, chlorpheniramine or a salt thereof such as the maleate, cyproheptadine or a salt thereof such as the hydrochloride, dexbrompheniramine or a salt thereof such as the maleate, dexchlorpheniramine or a salt thereof such as the maleate, diphenhydramine or a salt thereof such as the hydrochloride, doxylamine or a salt thereof such as the succinate, phenidamine or a salt thereof such as the tartrate, promethazine or a salt thereof such as the hydrochloride, pyrilamine or a salt thereof such as the maleate, pyrilamine or a salt thereof such as the tannate, tripelennamine or a salt thereof such as the hydrochloride, tripolidine or a salt thereof such as the hydrochloride, cetirizine or a salt thereof such as the hydrochloride, cinnarizine, mequitazine, dcivastine.

The amount of azatadine in the form of maleate in a unit dose may be in the range 1-2 mg, preferably 1 mg. The amount of bromodiphenhydramine in the form of hydrochloride in a unit dose may be 3.75 mg. The amount of brompheniramine in the form of maleate in a unit dose may be in the range 4-12 mg, preferably 4, 8 or 12 mg. The amount of carbinoxamine in the form of maleate in a unit dose may be 4 mg. The amount of chlorpheniramine in the form of maleate in a unit dose may be in the range 2-12 mg, preferably 4, 8 or 12 mg. The amount of dexbrompheniramine in the form of maleate in a unit dose may be 6 mg. The amount of dexchlorpheniramine in the form of maleate in a unit dose may be in the range of 2-6 mg, preferably 2, 4 or 6 mg. The amount of diphenhydramine in the form of hydrochloride in a unit dose may be in the range of 12.5 to 200 mg, preferably 12.5-50 mg, more preferably 12.5, 25 or 50 mg. The amount of doxylamine in the form of succinate in a unit dose may be in the range 7.5-10 mg, preferably 7.5 or 10 mg. The amount of phenidamine in the form of tartrate in a unit dose may be in the range 5-10 mg, preferably 5 or 10 mg. The amount of promethazine in the form of hydrochloride in a unit dose may be in the range 1.5-6 mg. The amount of pyrilamine in the form of maleate in a unit dose may be 12.5 mg. The amount of pyrilamine in the form of tannate in a unit dose may be 12.5 mg. The amount of tripelennamine in the form of hydrochloride in a unit dose may be in the range 25-50 mg, preferably 25, 37.5 or 50 mg. The amount of triprolidine in the form of hydrochloride in a unit dose may be in the range 1-2.5 mg, preferably 1.25-2.5 mg, most preferably 1.25 mg. The amount of cetirizine in a unit dose may be in the range 5-10 mg, preferably 5 mg or 10 mg. The amount of cinnarizine in a unit dose may be in the range of 15-75 mg, preferably 15 mg or 75 mg. The amount of mequitazine in a unit dose may be in the range 5-10 mg, preferably 5 mg or 10 mg. The amount of acrivastine in a unit dose may be 3-20 mg, preferably 5-10 mg, most preferably around 8 mg.

Suitable antiallergy agents include astemizole, clemastine or a salt thereof such as the hydrogen fumerate, loratadine, terfenodine.

The amount of astemizole in a unit dose may be in the range 0.5-200 mg, preferably 1-100 mg, most preferably 2, 5, 10, 20 or 40 mg. The amount of clemastine in the form of its hydrogen fumerate in a unit dose may be in the range 0.01-200 mg, preferably 0.1-10 mg, most preferably 0.2, 0.4, 0.6, 1.2 or 2.4 mg. The amount of loratadine in a unit dose may be in the range 0.5-200 mg, preferably 1-100 mg, most preferably 2, 5, 10, 20 or 40 mg. The amount of terfenadine in a unit dose may be in the range 5-1000 mg, preferably 10-600 mg, most preferably 20, 40, 60, 100 or 200 mg.

Suitable antacids include aluminium glycinate, aluminium hydroxide gel, aluminium phosphate gel, dried aluminium phosphate gel, calcium carbonate, charcoal, hydrotalcite, light kaolin, magnesium carbonate, magnesium hydroxide, magnesium oxide, magnesium trisilicate, sodium bicarbonate.

The amount of aluminium glycinate in a unit dose may be in the range 0.1-10 g, preferably 0.1-5g, most preferably 0.2, 0.5, 1 or 2 g. The amount of aluminium hydroxide gel in a unit dose may be in the range 1-50 ml, preferably 2-30 ml, most preferably 5, 7.5, 10, 15 or 30 ml. The amount of aluminium phosphate gel in a unit dose may be in the range 0.5-100 ml, preferably 1-50 ml, most preferably 2, 5, 10, 15 or 30 ml. The amount of dried aluminium phosphate gel in a unit dose may be in the range 50-5000 mg, preferably 100-2000 mg, most preferably 200, 400, 800 or 1600 mg. The amount of calcium carbonate in a unit dose may be in the range 0.1-30 g, preferably 0.5-10 g, most preferably 0.5, 1, 2 or 5 g. The amount of charcoal in a unit dose may be in the range 1-200g, preferably 1-100 g, most preferably 2, 4, 8, 16 or 50 g. The amount of hydrotalcite in a unit dose may be in the range 0.1-10 g, preferably 0.2-5 g, most preferably 0.5, 1 or 2 g. The amount of light kaolin in a unit dose may be in the range 10 mg-100 g, preferably 100 mg-75 g, most preferably 1, 10, 15, 20, 50 or 75 g. The amount of magnesium carbonate in a unit dose may be in the range 50 mg-10 g, preferably 50 mg-5 g, most preferably 100, 200 or 500 mg. The amount of magnesium hydroxide in a unit dose may be in the range 100 mg-10 g, preferably 100 mg-5 g, most preferably 100, 250, 500 or 750 mg. The amount of magnesium oxide in a unit dose may be in the range 100 mg-10 g, preferably 100 mg-5 g, most preferably 100, 250, 500 or 750 mg. The amount of sodium bicarbonate in a unit dose may be in the range 0.1-50 g, preferably 0.5-25 g, most preferably 0.5, 1, 2, 5 or 10 g.

Suitable antireflux agents include simethicone and sodium alginate.

The amount of simethicone in a unit dose may be in the range 5-1000 mg, preferably 10-500 mg, most preferably 25, 40, 50, 60, 100 or 200 mg. The amount of sodium alginate in a unit dose may be in the range 50 mg-10 g, preferably 75 mg-5 g, most preferably 100, 250, 500 or 1 g.

Suitable antiulcer agents include bismuth subsalicylate, H₂ receptor antagonists such as cimetidine, famotidine, ranitidine and nizatidine and proton pump inhibitors such as omeprazole, pantoprazole and lansoprazole.

The amount of bismuth subsalicylate in a unit dose may be in the range 250-2000 mg, preferably 50-1500 mg, most preferably 75, 150, 300, 600 or 1000 mg. The amount of cimetidine in a unit dose may be in the range 10 mg-5 g, preferably 50 mg-2 g, most preferably 100, 200 or 400 mg. The amount of famotidine in a unit dose may be in the range 10-80 mg, preferably 20 or 40 mg. The amount of ranitidine in a unit dose may be in the range 100-600 mg, preferably 300-600 mg, most preferably 300 or 600 mg. The amount of nizatidine in a unit dose may be 50 to 500 mg, preferably 100 to 400 mg, more preferably 150 to 300 mg. The amount of omeprazole in a unit dose may be 5 to 50 mg, preferably 10 to 40 mg, more preferably 10, 20 or 40 mg. The amount of pantoprazole in a unit dose may be 10 to 50 mg, preferably 15 to 45 mg, more preferably 20 to 40 mg. The amount of lansoprazole in a unit dose may be 5 to 50 mg, preferably 10 to 40 mg, more preferably 15 or 30 mg.

Suitable antidiarrhoeal agents include loperamide or a salt thereof, such as the hydrochloride, methylcellulose, diphenoxylate and morphine or a salt thereof, such as the hydrochloride.

The amount of loperamide in the for of its hydrochloride in a unit dose may be in the range 0.1-50 mg, preferably 0.5-20 mg, most preferably 1, 2, 4 or 8 mg. The amount of methylcellulose in a unit dose may be in the range 20 mg-5 g, preferably 50 mg-4 g, most preferably 100, 200, 500 mg, 1 or 2 g. The amount of diphenoxylate in the for of its hydrochloride in a unit dose may be 1-10 mg, preferably 2-5 mg, more preferably 2.5 mg. The amount of morphine in the for of its hydrochloride in a unit dose may be in the range 20-4000 µg, preferably 50-2000µg, most preferably 100, 200, 400, 800 or 1600 µg.

Suitable laxatives include agar, aloin, bisacodyl, ispaghula husk, lactulose, phenolphthalein and senna extract (including sennosides A + B).

The amount of agar in a unit dose may be in the range 1-200 mg, preferably 2-100 mg, most preferably 2-5, 5, 10, 20 or 50 mg. The amount of aloin in a unit dose may be in the range 1-200 mg, preferably 2-100 mg, most preferably 5, 10, 15 or 30 mg. The amount of bisacodyl in a unit dose may be in the range 0.1-100 mg, preferably 0.5-50 mg, most preferably 1, 2, 5, 10 or 20 mg. The amount of ispaghula husk in a unit dose may be in the range 100 mg-50 g, preferably 500 mg-25 g, most preferably 1, 2, 3, 5 or 10 g. The amount of lactulose in a unit dose may be in the range 100 mg-50 g, preferably 500 mg-30 g, most preferably 1, 2, 5, 10 or 15 g. The amount of phenolphthalein in a unit dose may be in the range 1-5000 mg, preferably 5-4000 mg, most preferably 7.5, 15, 30, 60, 100, 200 or 300 mg. The amount of senna extract (including sennosides A+B) in a unit dose may be in the range 0.5-100 mg, preferably 1-50 mg, most preferably 2.5, 5, 7.5, 10, 15 or 30 mg.

Suitable antiemetics include dimenhydrinate, metoclopromide or a salt thereof such as the hydrochloride, domperidone or a salt thereof such as the maleate, buclizine, cyclizine, prochlorperazine or a salt thereof such as the maleate, ipecacuanha, squill.

The amount of ipecacuanha in a unit dose may be in the range 25-100 mg. The amount of squill in a unit dose may be in the range 60-200 mg. The amount of domperidone may be in the range 5-50 mg, preferably 5, 10, 15, 20, 25, 30, 40 or 50 mg. The amount of buclizine in a unit dose may be in the range 2-100 mg, preferably 5-50 mg, more preferably 6.25, 13.5, 25, preferably 5-50 mg, more preferably 6.25, 13.5, 25 or 50 mg. The amount of cyclizine in a unit dose may be in the range 1-50 mg, preferably 2-30 mg, more preferably 5, 7.5, 10, 15, 20 or 25 mg. The amount of metoclopromide in a unit dose may be in the range 2-30 mg, preferably 5, 10, 15 or 30 mg. The amount of dimenhydrinate in a unit dose may be in the range 5-50 mg, preferably 25 mg. The amount of prochlorperazine in a unit dose may be in the range 3-25 mg, preferably 3 mg or 5 mg. If pharmaceutically effective salts of the above compounds are used then the amount of salt should be increased to give a dose of the free medicament corresponding to the figures given above.

Suitable agents to counter motion sickness include cinnarizine, dimenhydrinate, hyoscine or a salt thereof such as the hydrobromide and meclozine or a salt thereof such as the hydrochloride.

The amount of cinnarizine in a unit dose may be in the range 0.5-200 mg, preferably 1-100 mg, most preferably 5, 10, 20, 40 or 60 mg. The amount of dimenhydrinate in a unit dose may be in the range 1-500 mg, preferably 5-300 mg, most preferably 10, 20, 50, 100 or 250 mg. The amount of hyoscine hydrobromide in a unit dose may be in the range 0.01-1 mg, preferably 0.05-0.5 mg, most preferably 0.05, 0.1, 0.2, 0.3 or 0.5 mg. The amount of meclozine hydrochloride in a unit dose may be in the range 0.5-200 mg, preferably 1-100 mg, more preferably 2, 5, 10, 20 or 40 mg.

Suitable antiviral agents include aciclovir. The amount of aciclovir in a unit dose may be in the range 100 to 1000 mg, preferably 200 to 800 mg.

Suitable antifungal agents include fluconazole and terbinafine. The amount of fluconazole in a unit dose may be in the range 50-200 mg, preferably 50 mg or 200 mg. The amount of terbinafine may be in the range 250-500 mg, preferably 250 mg.

Suitable antibacterial agents include erythromycin and fusidic acid and salts thereof such as the sodium salt. The amount of erythromycin in a unit dose may be in the range 125-500 mg, preferably 125 mg, 250 mg or 500 mg. The amount of fusidic acid in a unit dose may be in the range 250-500 mg, preferably 250 mg.

Suitable diuretics include frusemide. The amount of frusemide in a unit dose may be in the range 20-80 mg, preferably 20, 40 or 80 mg.

Suitable anti-asthmatic agents include ketotifen. The amount of ketotifen in a unit dose may be in the range 1-4 mg, preferably 1 mg or 2 mg.

Suitable anti-migraine agents include sumatriptan. The amount of sumatriptan in a unit dose may be in the range 20-100 mg, preferably 20, 50 or 100 mg.

Suitable vitamins include A, B1, B2, B3, B5, B6, B12, C, D, E, folic acid, biotin, and K. Suitable minerals include calcium, phosphorus, iron, magnesium, zinc, iodine, copper, chloride, chromium, manganese, molybdenum, nickel, potassium, selenium, boron, tin and vanadium.

The medicament may be taste masked to further improve the taste profile of the pharmaceutical composition. The medicament may be taste masked using methods known in the art for example adding taste masking ingredients such as ethylcellulose, hydroxypropylmethylcellulose, methylethylcellulose, hydroxypropylcellulose, polyvinyl pyrrolidone, polyvinyl alcohol, mono glycerides, diglycerides, stearic acid, palmitic acid, gelatin, hydrogenated cotton seed oil and more generally any food grade polymer, starch, wax or fat. The taste masking agents may be used singly or in combination.

Preferably the unit dose of medicament is granulated optionally with other excipients. The other excipients may include taste masking agents, sweeteners, flavours, diluents, binders, lubricants.

Suitable sweeteners include acesulfame K, sodium saccharin, aspartame, sucrose. The amount of sweetener may be in the range 0.001% to 0.1%.

Suitable flavours are available commercially and the flavour may be enhanced by the addition of an acid such as citric acid, ascorbic acid, tartaric acid.

Suitable diluents include calcium phosphate (anhydrous and dihydrate), calcium sulphate, carboxymethylcellulose calcium, cellulose acetate, dexrates, dextrin, dextrose, fructose, glyceryl palmitostearate, hydrogenated vegetable oil, kaolin, lactitol, lactose, magnesium carbonate, magnesium oxide, maltitol, maltodextrin, maltose, microcrystalline cellulose, polymethacrylates, powdered cellulose, pregelatinised starch, silicified microcrystalline cellulose, sodium chloride, starch, sucrose, sugar, talc, xylitol. One or more diluents may be used. The amount of diluent may be in the range 10-98% w/w.

Suitable binders include acacia, alginic acid, carboxymethylcellulose, cellulose, dextrin, ethylcellulose, gelatin, glucose, guar gum, hydrogenated vegetable oil, hydroxyethylcellulose, hydroxypropylmethylcellulose, liquid glucose, magnesium aluminium silicate, maltodextrin, methylcellulose, polyethylene oxide, polymethacrylates, povidone, sodium alginate, starch, vegetable oil and zein. One or more binders may be used. The amount of binder may be in the range 10-50% w/w.

Suitable lubricants include calcium stearate, canola oil, glyceryl palmitostearate, hydrogenated vegetable oil, magnesium stearate, mineral oil, poloxamer, polyethylene glycol, polyvinyl alcohol, sodium benzoate, sodium lauryl sulphate, sodium stearyl fumarate, stearic acid, talc and zinc stearate. One or more lubricants may be used. The lubricant may be in the range 0.01-10% w/w.

The granules may be formed by methods known by the skilled man for example wet granulation or dry granulation.

The one or more second part of the pharmaceutical compositions of the present invention may be contained in a sealed container(s) prior to use. The sealed container may be any suitable container such as a sachet, pouch, ampoule or capsule made from a material which is compatible with the medicament and any other ingredients which are present. The sealed container should be easy to open to enable all its contents to be poured from the container.

The first and one or more secondn parts of the pharmaceutical composition of the present invention are kept separate until just before use. When the patient requires a unit dose of the medicament to be administered the two parts of the pharmaceutical composition are mixed and swallowed by the patient. To mix the parts, a suitable quantity of the liquid carrier is poured into a mixing vessel. The exact quantity of liquid carrier is not critical but is envisaged that an amount between 10 and 50 ml, preferably 15 to 30 ml will be used. The mixing vessel may be provided with markings indicating the suggested quantities but this is not essential. The sealed container holding the unit dose of medicament is then opened and the contents are poured into the liquid carrier. The liquid carrier and the contents of the sealed container should then be mixed in any suitable manner for example by stirring or shaking. The preferred method of mixing is by shaking. To facilitate this the mixing vessel may be provided with a removable lid which when fitted to the mixing vessel seals the mixing vessel enabling the vessel to be shaken to distribute the unit dose of medicament throughout the liquid carrier. It is envisaged that the mixed contents of the mixing vessel will be ingested by the patient soon after mixing. Because of this there will be no time for any adverse interaction between the medicament and any of the excipients used in the liquid carrier as can sometimes occur in the prior art liquid compositions on storage.

A further aspect of the present invention also provides a kit of parts for preparing a liquid unit dosage form of a medicament or medicaments comprising a range of first containers containing differently flavoured liquid carriers, one or more second sealed containers containing a solid or liquid unit dose of the medicament or medicaments and a separate mixing vessel into which a quantity of selected liquid carrier and the unit dose of the medicament or medicaments can be introduced and mixed. Preferably the mixing vessel is provided with a lid which seals the container enabling the liquid carrier and the unit dose of medicament to be mixed by shaking the sealed mixing vessel. The mixed pharmaceutical composition may then be ingested by the patient from the mixing vessel.

The pharmaceutical compositions of the present invention described herein have several advantages over the known liquid dosage forms.
1. Because the liquid carrier and the second or more part incorporating the medicament are mixed immediately prior to administration, there can be no interactions between the medicament and any of the excipients as sometimes occurs in the known compositions on storage because the medicament and excipients are in contact for long periods of time. Because of this possible instability of the known compositions, each one of them must be subjected to time consuming and expensive stability testing. In the present invention each flavoured variety of the liquid carrier must be tested but only once. There is no requirement to test the liquid carrier every time a composition containing a new medicament is required.
2. The medicaments are stored in separate containers in solid or liquid form. The possibility of interactions with other excipients in the known liquid compositions does not occur with the present invention. The contents of the container must, of course, undergo stability testing but this will be much simpler than testing the known liquid compositions.
3. The patient can choose the flavour of the liquid carrier for his or her medicine which is acceptable to him or her. This will improve patient compliance with the dosing regime.
4. The amount of wastage is reduced as the liquid carrier can be stored until it is needed again for use with a different medicament to treat different symptoms in the patient.
5. The present invention makes it easy to give combinations of medicaments which are specifically directed at the symptoms of any particular patient, by mixing the liquid carrier with unit doses of more than one medicament. In the known liquid compositions containing combinations of medicaments is selected by the manufacturer and it may not be the combination which is actually needed by any particular patient.
6. Taste will improve due to the bitterness of active ingredients being suppressed by tastemasking, therefore having a positive effect on the overall taste of the formulation.
7. The present invention avoids the present undesirable practice of storing a number of partly used liquid compositions which will be used again if the illness recurs. Using the present invention, any unconsumed liquid carrier can be used to treat a different illness by mixing into the liquid carrier a different solid unit dose of medicament.

The invention will be illustrated by the following Examples which are given by way of example only.

### Example 1 ― Sugar-free liquid carrier

| Ingredient | % weight or v/v |
|---|---|
| | |
| Glycerol | 10 v |
| Maltitol solution | 30 v |
| Sodium benzoate | 0.2 w |
| Citric acid | 0.2 w |
| Acesulfame K | 0.025 w |
| Sodium saccharin | 0.0125 w |
| Liquid flavour | 0.1 v |
| Purified water | to 100 v |

The liquid carrier uses the sweetness and mouthfeel of glycerol and maltitol to give a good mouth-feel taste. Thickener systems may be used to give a thicker syrup.

### Example 2 ― Liquid carrier containing sugar

| Ingredient | % weight or v/v |
|---|---|
| Glycerol | 10 v |
| Liquid sugar demin | 40 v |
| Sodium benzoate | 0.2 w |
| Citric acid | 0.2 w |
| Liquid flavour | 0.1 v |
| Purified water | to 100 v |

The base uses the sweetness and mouth-feel of glycerol and liquid sugar to give a good mouth-feel. Thickener systems may be used to give a thicker syrup.

### Example 3

| Ingredient | mg/sachet |
|---|---|
| Taste masked paracetamol | 555.55 |
| Total | 555.55 |

Taste masked paracetamol is calculated as being 90% of paracetamol and 10% of ethyl cellulose as the taste masking ingredient. Therefore the actual dose of paracetamol is 500 mg.

The powdered taste masked medicament is sealed within a sachet. When the medicament is to be administered to the patient, the contents of the sachet are added to a quantity (preferably 5 to 30 ml) of the liquid carrier of either Example 1 or Example 2 and mixed by shaking. The resulting liquid dosage form is then taken by the patients.

### Example 4

| Ingredient | mg/sachet |
|---|---|
| Taste masked ibuprofen | 285.71 |
| Xylitol | 274.29 |
| Total | 560 |

Taste masked ibuprofen is based on 70% of ibuprofen and 30% mono and diglycerides of fatty acids as the taste masking ingredient. Therefore the actual dose of ibuprofen is 200 mg.

The powdered taste masked medicament is sealed within a sachet. When the medicament is to be administered to the patient, the contents of the sachet are added to a quantity (preferably 5 to 30 ml) of the liquid carrier of either Example 1 or Example 2 and mixed by shaking. The resulting liquid dosage form is then taken by the patients.

### Example 5

| Ingredient | mg/sachet |
|---|---|
| Taste masked aspirin | 375 |
| Xylitol | 190 |
| Total | 565 |

Taste masked aspirin is 80% aspirin, 20% ethyl cellulose as the taste masking agent. Therefore sachet contains 300 mg of the active ingredient.

The powdered taste masked medicament is sealed within a sachet. When the medicament is to be administered to the patient, the contents of the sachet are added to a quantity (preferably 5 to 30 ml) of the liquid carrier of either Example 1 or Example 2 and mixed by shaking. The resulting liquid dosage form is then taken by the patients.

### Example 6

| Ingredient | mg/sachet |
|---|---|
| Taste masked paracetamol | 277.8 |
| Lactose | 222.2 |
| Total | 500 |

Taste masked paracetamol is 90% paracetamol, 10% ethyl cellulose as the taste masking agent. Therefore a sachet contains 250 mg of the active ingredient.

The powdered taste masked medicament is sealed within a sachet. When the medicament is to be administered to the patient, the contents of the sachet are added to a quantity (preferably 5 to 30 ml) of the liquid carrier of either Example 1 or Example 2 and mixed by shaking. The resulting liquid dosage form is then taken by the patients.

### Example 7

The efficacy of the compositions of the present invention were demonstrated in the following way. The formulation of Example 6 was mixed with the sugar free liquid carrier of Example 1 and an expert panel were asked to compare the taste with that of a widely-used paediatric pain relief oral suspension containing paracetamol. 87% of the panel preferred the taste of the composition according to the present application.

## Claims

1. A method of preparing a liquid unit dosage form of a medicament or medicaments comprising;
providing a range of first containers containing different liquid carriers,
providing one or more second, sealed containers containing a solid or liquid unit dose of the medicament or medicaments,
pouring a quantity of a selected one of the liquid carriers into a mixing vessel, opening the one or more second, sealed containers,
pouring the unit dose of the medicament or medicaments from the one or more second containers into the mixing vessel, and
allowing or causing the liquid carrier and the unit dose of medicament or medicaments to become mixed in the mixing vessel.

2. A method as claimed in Claim 1 in which the liquid carrier comprises water, glycerol and/or sorbitol, and optionally a natural or synthetic sweetener and/or thickening agent.

3. A method as claimed in Claim 1 or Claim 2, wherein the liquid carrier comprises a patient-acceptable flavouring and optionally an organic acid flavouring enhancer.

4. A method as claimed in any one of claims 1 to 3, wherein the mixing vessel is provided with a lid which seals the mixing vessel allowing the contents to be mixed by shaking the sealed vessel.

5. A kit of parts for preparing a liquid unit dosage form of a medicament or medicaments , the kit comprising;
a range of first containers containing differently flavoured liquid carriers,
one or more second, sealed containers containing a solid or liquid unit dose of the medicament or medicaments, and
a separate mixing vessel into which a quantity of selected liquid carrier and the unit dose of the medicament or medicaments can be introduced and mixed.

6. A kit as claimed in Claim 5 in which the liquid carrier comprises water, glycerol and/or sorbitol, and optionally a natural or synthetic sweetener and/or thickening agent.

7. A kit as claimed in Claim 5 or Claim 6, wherein the liquid carrier comprises a patient-acceptable flavouring and optionally an organic acid flavouring enhancer.

8. A kit as claimed in any one of claims 5 to 7, wherein the mixing vessel is provided with a lid which seals the mixing vessel allowing the contents to be mixed by shaking the sealed vessel.

## Patentansprüche

1. Ein Verfahren zur Herstellung einer flüssigen Dosierungseinheit einer Mischung eines Medikaments oder von Medikamenten, welches umfasst:
Zur-Verfügung-Stellung einer Reihe von ersten Behältern, die verschiedene Flüssigkeitsträger beinhalten,
Zur-Verfügung-Stellung eines oder mehrerer zweiter dicht verschlossener Behälter, die eine feste oder flüssige Dosierungseinheit des Medikaments oder von Medikamenten beinhalten,
Ausgießen einer Menge eines ausgewählten Flüssigkeitsträgers in ein Mischgefäß,
Öffnen des einen oder der mehreren zweiten dicht verschlossenen Behälter, Ausgießen der Dosierungseinheit des Medikaments oder der Medikamente von dem einen oder den mehreren zweiten Behältern in das Mischgefäß, und
Gestatten und Veranlassen des Flüssigkeitsträgers und der Dosierungseinheit des Medikaments oder der Medikamente, in dem Mischgefäß vermischt zu werden.

2. Ein Verfahren nach Anspruch 1, bei dem der Flüssigkeitsträger Wasser, Glycerol bzw. Sorbitol und optional einen natürlichen oder synthetischen Zuckeraustauschstoff bzw. ein Verdickungsmittel umfasst.

3. Ein Verfahren nach nach Anspruch 1 oder Anspruch 2, wobei der Flüssigkeitsträger einen für den Patienten angenehmen aromatischen Geschmacksstoff und optional einen auf einer organischen Säure basierenden Geschmacksverstärker umfasst.

4. Ein Verfahren nach einem der Ansprüche 1 bis 3, wobei das Mischgefäß mit einem Deckel ausgestattet ist, der das Mischgefäß dicht verschließt, wobei es den Inhalten gestattet wird, durch Schütteln des dicht verschlossenen Gefäßes vermischt zu werden.

5. Ein Set von Teilen zur Herstellung einer flüssigen Dosierungseinheit einer Mischung eines Medikaments oder von Medikamenten, wobei das Set umfasst;
eine Reihe von ersten Behältern, die Flüssigkeitsträger mit verschiedenen Geschmacksrichtungen beinhalten,
ein oder mehrere zweite dicht verschlossene Behälter, die eine feste oder flüssige Dosierungseinheit des Medikaments oder von Medikamenten beinhalten, und
ein gesondertes Mischgefäß, in das eine Menge eines ausgewählten Flüssigkeitsträgers und eine Dosierungseinheit eines Medikaments oder von Medikamenten zugeführt werden kann und dort vermischt werden kann.

6. Ein Set nach Anspruch 5, bei dem der Flüssigkeitsträger Wasser, Glycerol bzw. Sorbitol und optional einen natürlichen oder synthetischen Zuckeraustauschstoff bzw. ein Verdickungsmittel umfasst.

7. Ein Set nach Anspruch 5 oder Anspruch 6, wobei der Flüssigkeitsträger einen für den Patienten angenehmen aromatischen Geschmacksstoff und optional einen auf einer organischen Säure basierenden Geschmacksverstärker umfasst.

8. Ein Set nach einem der Ansprüche 5 bis 7, wobei das Mischgefäß mit einem Deckel ausgestattet ist, der das Mischgefäß dicht verschließt, wobei es den Inhalten gestattet wird, durch Schütteln des dicht verschlossenen Gefäßes vermischt zu werden.

## Revendications

1. Une méthode de préparation pour une unité de dosage sous forme liquide d'un ou de plusieurs médicaments consistant :
en une première série de récipients contenant différents porteurs liquides,
en un ou plusieurs deuxièmes récipients hermétiques contenant une unité de dosage solide ou liquide du ou des médicament(s),
à verser une quantité de porteur liquide sélectionné dans un réservoir de mélange,
à ouvrir le ou les seconds récipients hermétiques,
à verser l'unité de dosage du ou des médicaments depuis le ou les deuxièmes récipients dans le réservoir de mélange, et
à permettre ou à faire en sorte que le porteur liquide et l'unité de dosage du médicament ou des médicaments se mélangent à l'intérieur du réservoir.

2. Une méthode selon la revendication 1, dans laquelle le porteur liquide comprend de l'eau, du glycérol et/ou du sorbitol, et, de manière facultative, un édulcorant naturel ou synthétique et/ou un agent épaississant.

3. Une méthode selon la revendication 1 ou la revendication 2, dans laquelle le porteur liquide comprend un aromatisant acceptable par le patient et, de manière facultative, un acide organique exhausteur de goût.

4. Une méthode selon n'importe laquelle des revendications allant de 1 à 3, dans laquelle le réservoir de mélange est fourni avec un couvercle qui ferme le réservoir de mélange permettant au contenu d'être secoué et mélangé par le réservoir hermétique.

5. Un jeu d'éléments servant à préparer une unité de dosage sous forme liquide d'un ou de plusieurs médicaments, le jeu comprenant :
une première série de récipients contenant des porteurs liquides aux différentes saveurs ;
un ou plusieurs deuxièmes récipients hermétiques contenant une unité de dosage solide ou liquide du ou des médicament(s), et
un réservoir de mélange indépendant dans lequel il est possible d'introduire et de mélanger un porteur liquide sélectionné et une unité de dosage du ou des médicament(s).

6. Un jeu selon la revendication 5 dans lequel le porteur liquide comprend de l'eau, du glycérol et/ou du sorbitol, et, de manière facultative, un édulcorant naturel ou synthétique et/ou un agent épaississant.

7. Un jeu selon la revendication 5 ou la revendication 6, dans lequel le porteur liquide comprend un aromatisant acceptable par le patient et, de manière facultative, un acide organique exhausteur de goût.

8. Un jeu selon n'importe laquelle des revendications allant de 5 à 7, dans lequel le réservoir de mélange est fourni avec un couvercle qui ferme le réservoir de mélange permettant au contenu d'être secoué et mélangé par le réservoir hermétique.
